# EUROPEAN PATENT SPECIFICATION

(11) **EP 1 654 262 B1**
(45) Date of publication and mention of the grant of the patent: **25.10.2006**
(21) Application number: 04764070.1
(22) Date of filing: 11.08.2004
(51) Int. Cl.: C07D 471/04, A61K 31/437, A61P 29/00

(54) **7-AZAINDOLE DERIVATIVES AS COX2 INHIBITORS**
7-AZAINDOLDERIVATE ALS COX2-INHIBITOREN
DERIVES DU 7-AZAINDOLE UTILISES COMME INHIBITEURS DE COX-2

(30) Priority: 13.08.2003 GB 0319037
(43) Date of publication of application: 10.05.2006
(73) Proprietor: GLAXO GROUP LIMITED, Greenford, Middlesex UB6 ONN (GB)
(72) Inventor: BESWICK, Paul c/o GlaxoSmithKline, Harlow Essex CM19 5AW (GB); GLEAVE, Robert c/o GlaxoSmithKline, Harlow Essex CM19 5AW (GB); SWARBRICK, Martin c/o GlaxoSmithKline, Harlow Essex CM19 5AW (GB)
(74) Representative: Cooke, Tracey
(86) International application number: PCT/EP2004/009073
(87) International publication number: WO 2005/016924

(56) References cited:
- WO-A-99/61436
- US-B1- 6 300 363

## Description

This invention relates to 7-azaindole derivatives, to processes for their preparation, to pharmaceutical compositions containing them and to their use in medicine.

The enzyme cyclooxygenase (COX) has recently been discovered to exist in two isoforms, COX-1 and COX-2. COX-1 corresponds to the originally identified constitutive enzyme while COX-2 is rapidly and readily inducible by a number of agents including mitogens, endotoxin, hormones, cytokines and growth factors. Prostaglandins generated by the action of COX have both physiological and pathological roles. It is generally believed that COX-1 is largely responsible for the important physiological functions such as maintenance of gastrointestinal integrity and renal blood flow. In contrast the inducible form, COX-2, is believed to be largely responsible for the pathological effects of prostaglandins where rapid induction of the enzyme occurs in response to such agents as inflammatory agents, hormones, growth factors and cytokines. A selective inhibitor of COX-2 would therefore have anti-inflammatory, anti-pyretic and analgesic properties, without the potential side effects associated with inhibition of COX-1. We have now found a novel group of compounds which are both potent and selective inhibitors of COX-2.

The invention thus provides a compound of formula (I) 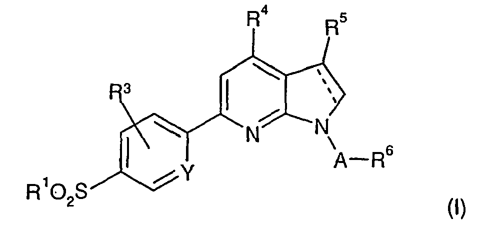 or a pharmaceutically acceptable salt thereof in which:
Y is selected from the group consisting of CH or nitrogen;
R¹ is selected from the group consisting of C₁₋₆alkyl, NH₂ and R²CONH;
R² is selected from the group consisting of H, C₁₋₆alkyl, C₁₋₆alkoxy,
C₁₋₆alkylOC₁₋₆alkyl, phenyl, HO₂CC₁₋₆alkyl, C₁₋₆alkylOCOC₁₋₆alkyl, C₁₋₆alkylOCO, H₂NC₁₋₆alkyl, C₁₋₆alkylOCONHC₁₋₆alkyl and C₁₋₆alkylCONHC₁₋₆alkyl;
R³ is selected from the group consisting of H and halogen;
R⁴ is selected from the group consisting of H, C₁₋₅alkyl, and C₁₋₂alkyl substituted by one to five fluorine atoms;
R⁵ is selected from the group consisting of H, CHO, and C₁₋₆alkyl which is unsubstituted or is substituted one or more times by halogen or hydroxy;
A is (CH₂)ₙ or -SO₂-;
R⁶ is selected from the group consisting of C₁₋₆alkyl, C₄₋₈ cycloalkyl, phenyl and 6-membered heteroaryl, wherein the phenyl and 6-membered heteroaryl ring may be unsubstituted or substituted one or more times by halogen or C₁₋₆ alkyl; and
n is 0 to 3.

The term 'halogen' denotes fluorine, chlorine, bromine or iodine.

The term 'alkyl' as a group or part of a group denotes a straight or branched chain alkyl group, for example a methyl, ethyl, n-propyl, i-propyl, n-butyl, s-butyl or t-butyl group.

The term '6- membered heteroaryl' denotes a heteroaryl selected from the following:

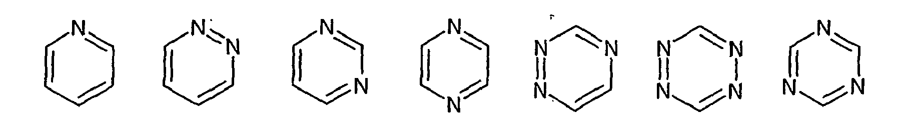

It is to be understood that the present invention encompasses all isomers of the compounds of formula (I) which are also potent and selective inhibitors of COX-2, and their pharmaceutically acceptable salts, including all geometric, tautomeric and optical forms, and mixtures thereof (e.g. racemic mixtures).

It will be appreciated that in some instances, compounds of the present invention may include a basic function such as an amino group as a substituent. Such basic functions may be used to form acid addition salts, in particular pharmaceutically acceptable salts. Pharmaceutically acceptable salts include those described by Berge, Bighley, and Monkhouse, *J. Pharm. Sci.,* 1977, 66_{;} 1-19. Such salts may be formed from inorganic and organic acids. Representative examples thereof include maleic, fumaric, benzoic, ascorbic, pamoic, succinic, bismethylenesalicylic, methanesulfonic, ethanedisulfonic, acetic, propionic, tartaric, salicylic, citric, gluconic, aspartic, stearic, palmitic, itaconic, glycolic, p-aminobenzoic, glutamic, taurocholic acid, benzenesulfonic, p-toluenesulfonic, hydrochloric, hydrobromic, sulfuric, cyclohexylsulfamic, phosphoric and nitric acids.

It will be appreciated that in some instances, compounds of the present invention may include a carboxy group as a substituent. Such carboxy groups may be used to form salts, in particular pharmaceutically acceptable salts. Pharmaceutically acceptable salts include those described by Berge, Bighley, and Monkhouse, *J*. *Pharm. Sci.,* 1977, **66**, 1-19. Preferred salts include alkali metal salts such as the sodium and potassium salts.

In one aspect the invention provides a compound of formula (IA) 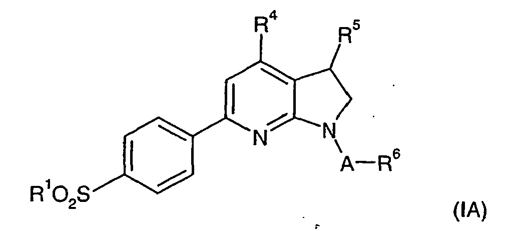 or a pharmaceutically acceptable salt thereof, in which all substituents are as for a compound of formula (I) as defined above.

In another aspect the invention provides a compound of formula (IB) 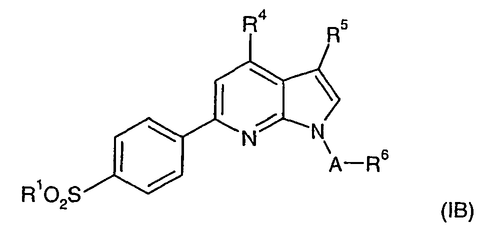 or a pharmaceutically acceptable salt thereof, in which all substituents are as for a compound of formula (I) as defined above.

In another aspect of the invention R¹ is C₁₋₆alkyl, such as C₁₋₃alkyl.

Representative examples of R¹ include CH₃.

In another aspect of the invention R² is selected from the group consisting of C₁₋₆alkyl (e.g. ethyl), phenyl and aminomethyl.

In another aspect of the invention R⁴ is H, CHF₂, CH₂F, CF₃ or C₁₋₄alkyl.

Representative examples of R⁴ include H.

In another aspect of the invention, R⁵ is H, C₁₋₄alkyl, -CHO, or -(CH₂)ₙCH₂OH.

Representative examples of R⁵ include H, methyl, s-butyl, CHO and CH₂OH.

In another aspect of the invention n is 0 or 1.

In another aspect of the invention, R⁶ is C₃₋₅alkyl, cyclohexyl, pyridyl optionally substituted by C₁₋₃alkyl, or phenyl optionally substituted by halogen.

Representative examples of R⁶ include cyclohexyl, phenyl, 2-methylpyrid-3-yl, pent-3-yl, propyl, 4-fluorophenyl, pyrid-2-yl, pyrid-3-yl, pyrid-4-yl, 6-methylpyrid-3-yl, and 4-chlorophenyl.

In another aspect of the invention n is 1 when R⁶ is pyridyl.

In another aspect the invention provides a group of compounds of formula (IB) wherein R¹ is C₁₋₃alkyl, R⁴ is H, CHF₂, CH₂F, CF₃ or C₁₋₄alkyl, R⁵ is H, C₁₋₄alkyl,-CHO, or -CH₂OH, n is 1, and R⁶ is C₃₋₅alkyl, cyclohexyl, pyridyl optionally substituted by C₁₋₃alkyl, or phenyl optionally substituted by halogen.

In another aspect the invention provides a group of compounds of formula (IB) wherein R¹ is C₁₋₃alkyl, R⁴ is H, CHF₂, CH₂F, CF₃or C₁₋₄alkyl, R⁵ is H, C₁₋₄alkyl, -CHO, or -CH₂OH, n is 0, and R⁶ is phenyl optionally substituted by halogen.

In another aspect the invention provides a group of compounds of formula (IB) wherein R¹ is CH₃, R³ is H, R⁴ is H, R⁵ is H, C₁₋₄alkyl, -CHO, or -CH₂OH, A is (CH₂)ₙ and n is 1, and R⁶ is C₃₋₅alkyl, cyclohexyl, pyridyl optionally substituted by CH₃, or phenyl optionally substituted by chloro.

In another aspect the invention provides a group of compounds of formula (IB) wherein R¹ is CH₃, R³ is H, R⁴ is H, R⁵ is H, A is (CH₂)ₙ and n is 0, and R⁶ is phenyl optionally substituted by fluoro.

It is to be understood that the invention covers all combinations of particular aspects of the invention as described hereinabove.

Since the compounds of the present invention, in particular compounds of formula (I), are intended for use in pharmaceutical compositions, it will be understood that they are each provided in substantially pure form, for example at least 50% pure, more suitably at least 75% pure and preferably at least 95% pure (% are on a wt/wt basis). Impure preparations of the compound of formula (I) may be used for preparing the more pure forms used in pharmaceutical compositions. Although the purity of intermediate compounds of the present invention is less critical, it will be readily understood that the substantially pure form is preferred as for the compounds of formula (I). Preferably, whenever possible, the compounds of the present invention are available in crystalline form.

When some of the compounds of this invention are allowed to crystallise or are recrysallised from organic solvents, solvent of recrystallisation may be present in the crystalline product. This invention includes within its scope such solvates. Similarly, some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation. In addition, different crystallisation conditions may lead to the formation of different polymorphic forms of crystalline products. This invention includes within its scope all the polymorphic forms of the compounds of formula (I).

Compounds of the invention are potent and selective inhibitors of COX-2. This activity is illustrated by their ability to selectively inhibit COX-2 over COX-1.

In view of their selective COX-2 inhibitory activity, the compounds of the present invention are of interest for use in human and veterinary medicine, particularly in the treatment of the pain (both chronic and acute), fever and inflammation of a variety of conditions and diseases mediated by selective inhibition of COX-2. Such conditions and diseases are well known in the art and include rheumatic fever; symptoms associated with influenza or other viral infections, such as the common cold; lower back and neck pain; headache; toothache; sprains and strains; myositis; sympathetically maintained pain; synovitis; arthritis, including rheumatoid arthritis; degenerative joint diseases, including osteoarthritis; gout and ankylosing spondylitis; tendinitis; bursitis; skin related conditions, such as psoriasis, eczema, burns and dermatitis; injuries, such as sports injuries and those arising from surgical and dental procedures.

The compounds of the invention are also useful for the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; non-specific lower back pain; multiple sclerosis pain; fibromyalgia; HIV-related neuropathy; neuralgia, such as post-herpetic neuralgia and trigeminal neuralgia; and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

The compounds of the invention are also useful for the treatment of other conditions mediated by selective inhibition of COX-2.

For example, the compounds of the invention inhibit cellular and neoplastic transformation and metastatic tumour growth and hence are useful in the treatment of certain cancerous diseases, such as colonic cancer and prostate cancer. The compounds of the invention are also useful in reducing the number of adenomatous colorectal polyps and thus reduce the risk of developing colon cancer. The compounds of the invention are also useful in the treatment of cancer associated with overexpression of HER-2/neu, in particular breast cancer.

Compounds of the invention also prevent neuronal injury by inhibiting the generation of neuronal free radicals (and hence oxidative stress) and therefore are of use in the treatment of stroke; epilepsy; and epileptic seizures (including grand mal, petit mal, myoclonic epilepsy and partial seizures).

Compounds of the invention also inhibit prostanoid-induced smooth muscle contraction and hence are of use in the treatment of dysmenorrhoea and premature labour.

Compounds of the invention are also useful in the treatment of liver disease, such as inflammatory liver disease, for example chronic viral hepatitis B, chronic viral hepatitis C, alcoholic liver injury, primary biliary cirrhosis, autoimmune hepatitis, nonalcoholic steatohepatitis and liver transplant rejection.

Compounds of the invention inhibit inflammatory processes and therefore are of use in the treatment of asthma, allergic rhinitis and respiratory distress syndrome; gastrointestinal conditions such as inflammatory bowel disease, Crohn's disease, gastritis, irritable bowel syndrome and ulcerative colitis; and the inflammation in such diseases as vascular disease, migraine, periarteritis nodosa, thyroiditis, aplastic anaemia, Hodgkin's disease, sclerodoma, type I diabetes, myasthenia gravis, multiple sclerosis, sorcoidosis, nephrotic syndrome, Bechet's syndrome, polymyositis, gingivitis, conjunctivitis and myocardial ischemia.

Compounds of the invention are also useful in the treatment of ophthalmic diseases such as retinitis, retinopathies, uveitis and of acute injury to the eye tissue.

Compounds of the invention are also useful for the treatment of cognitive disorders such as dementia, particularly degenerative dementia (including senile dementia, Alzheimer's disease, Pick's disease, Huntington's chorea, Parkinson's disease and Creutzfeldt-Jakob disease), and vascular dementia (including multi-infarct dementia), as well as dementia associated with intracranial space occupying lesions, trauma, infections and related conditions (including HIV infection), metabolism, toxins, anoxia and vitamin deficiency; and mild cognitive impairment associated with ageing, particularly Age Associated Memory Impairment.

Compounds of the invention are also useful in the treatment of disorders ameliorated by a gastroprokinetic agent. Disorders ameliorated by gastroprokinetic agents include ileus, for example post-operative ileus and ileus during sepsis; gastroesophageal reflux disease (GORD, or its synonym GERD); gastroparesis, such as diabetic gastroparesis; and other functional bowel disorders, such as non-ulcerative dyspepsia (NUD) and non-cardiac chest pain (NCCP).

According to a further aspect of the invention, we provide a compound of formula (I) for use in human or veterinary medicine.

According to some embodiments of the invention, we provide the use of a compound of formula (I) for the manufacture of a therapeutic agent for the treatment of a condition which is mediated by COX-2.

According to some embodiments of the invention, we provide the use of a compound of formula (I) for the manufacture of a therapeutic agent for the treatment of an inflammatory disorder.

It is to be understood that reference to treatment includes both treatment of established symptoms and prophylactic treatment, unless explicitly stated otherwise.

It is to be understood that all references to compounds of formula (I) specifically include references to compounds of formula (IA) and compounds of formula (IB), and each one of the examples hereto.

It will be appreciated that the compounds of the invention may advantageously be used in conjunction with one or more other therapeutic agents. Examples of suitable agents for adjunctive therapy include a 5HT₁ agonist, such as a triptan (e.g. sumatriptan or naratriptan); an adenosine A1 agonist; an EP ligand; an NMDA modulator, such as a glycine antagonist; a sodium channel blocker (e.g. lamotrigine); a substance P antagonist (e.g. an NK₁ antagonist); a cannabinoid; acetaminophen or phenacetin; a 5-lipoxygenase inhibitor; a leukotriene receptor antagonist; a DMARD (e.g. methotrexate); gabapentin and related compounds; a tricyclic antidepressant (e.g. amitryptilline); a neurone stabilising antiepileptic drug; a mono-aminergic uptake inhibitor (e.g. venlafaxine); a matrix metalloproteinase inhibitor; a nitric oxide synthase (NOS) inhibitor, such as an iNOS or an nNOS inhibitor; an inhibitor of the release, or action, of tumour necrosis factor α; an antibody therapy, such as a monoclonal antibody therapy; an antiviral agent, such as a nucleoside inhibitor (e.g. lamivudine) or an immune system modulator (e.g. interferon); an opioid analgesic; a local anaesthetic; a stimulant, including caffeine; an H₂-antagonist (e.g. ranitidine); a proton pump inhibitor (e.g. omeprazole); an antacid (e.g. aluminium or magnesium hydroxide; an antiflatulent (e.g. simethicone); a decongestant (e.g. phenylephrine, phenylpropanolamine, pseudoephedrine, oxymetazoline, epinephrine, naphazoline, xylometazoline, propylhexedrine, or levo-desoxyephedrine); an antitussive (e.g. codeine; hydrocodone, carmiphen, carbetapentane, or dextramethorphan); a diuretic; or a sedating or non-sedating antihistamine. It is to be understood that the present invention covers the use of a compound of formula (I) in combination with one or more other therapeutic agents.

The compounds of formula (I) are conveniently administered in the form of pharmaceutical compositions. Thus, in some embodiments of the invention, we provide a pharmaceutical composition comprising a compound of formula (I) adapted for use in human or veterinary medicine. Such compositions may conveniently be presented for use in conventional manner in admixture with one or more physiologically acceptable carriers or excipients.

As will be appreciated by the person skilled in the art the compounds of the invention may be milled using known milling procedures such as wet milling to obtain a particle size appropriate for tablet formation and for other formulation types. In particular, for those compounds which demonstrate poor bioavailability, finely divided (nanoparticulate) preparations of the compounds of the invention may be prepared by processes known in the art, for example see International Patent Application No. WO 02/00196 (SmithKline Beecham).

The compounds of formula (I) may be formulated for administration in any suitable manner. They may, for example, be formulated for topical administration or administration by inhalation or, more preferably, for oral, transdermal or parenteral administration. The pharmaceutical composition may be in a form such that it can effect controlled release of the compounds of formula (I).

For oral administration, the pharmaceutical composition may take the form of, for example, tablets (including sub-lingual tablets), capsules, powders, solutions, syrups or suspensions prepared by conventional means with acceptable excipients.

For transdermal administration, the pharmaceutical composition may be given in the form of a transdermal patch, such as a transdermal iontophoretic patch.

For parenteral administration, the pharmaceutical composition may be given as an injection or a continuous infusion (e.g. intravenously, intravascularly or subcutaneously). The compositions may take such forms as suspensions, solutions or emulsions in oily or aqueous vehicles and may contain formulatory agents such as suspending, stabilising and/or dispersing agents. For administration by injection these may take the form of a unit dose presentation or as a multidose presentation preferably with an added preservative.

Alternatively for parenteral administration the active ingredient may be in powder form for reconstitution with a suitable vehicle.

The compounds of the invention may also be formulated as a depot preparation. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

As stated above, the compounds of the invention may also be used in combination with other therapeutic agents. The invention thus provides, in a further aspect, a combination comprising a compound of formula (I) together with a further therapeutic agent.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) is used in combination with a second therapeutic agent active against the same disease state the dose of each compound may differ from that when the compound is used alone. Appropriate doses will be readily appreciated by those skilled in the art.

A proposed daily dosage of a compound of formula (I) for the treatment of man is 0.01mg/kg to 50mg/kg, such as 0.05mg/kg to 10mg/kg, e.g. 0.1mg/kg to 5mg/kg, which may be conveniently administered in 1 to 4 doses. The precise dose employed will depend on the age and condition of the patient and on the route of administration. Thus, for example, a daily dose of 0.25mg/kg to 10mg/kg may be suitable for systemic administration.

Compounds of formula (I) may be prepared by any method known in the art for the preparation of compounds of analogous structure.

Thus, compounds of formula (IA), where each of R⁴ and R⁵ is hydrogen may be prepared by a process which comprises:
reducing a compound of formula (III) 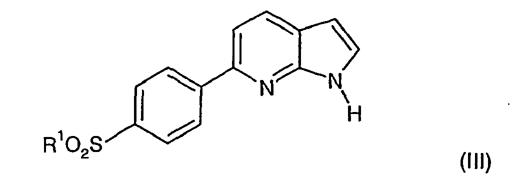
to form a compound of formula (VIII); 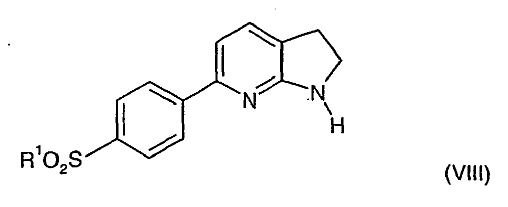
reacting said compound of formula (VIII) with a compound R⁶-A-X, or a protected derivative thereof, where X is a halogen, such as Cl, Br or I, or a sulfonate such as methanesulfonate, (4-methyl)benzenesulfonate or trifluoromethanesulfonate, and A and R⁶ are as hereinbefore defined; such as to produce a compound of formula (IA), wherein R⁴ and R⁵ are both hydrogen 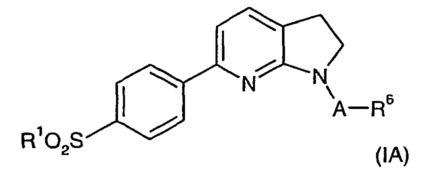
and thereafter and if necessary,
interconverting said compound of formula (IA) into another compound of formula (IA); and/or
deprotecting a protected derivative of compound of formula (IA).

The synthesis of a compound of formula (IA), wherein R⁴ and R⁵ are both hydrogen, is shown in Scheme 1 below, in which R¹, R⁶ and A are as defined in relation to formula (I) unless otherwise stated; X is a halogen, such as CI, Br or I, or a sulfonate, such as methanesulfonate, (4-methyl)benzenesulfonate or trifluoromethanesulfonate.

Referring to Scheme 1, compounds of formula (IA) may be prepared by the treatment of compounds of formula (VIII) with R⁶-A-X in the presence of a base, such as sodium hydride. The reaction is conveniently carried out in a solvent, such as DMF (N,N-dimethylformamide) and at between 0ºC and elevated temperature.

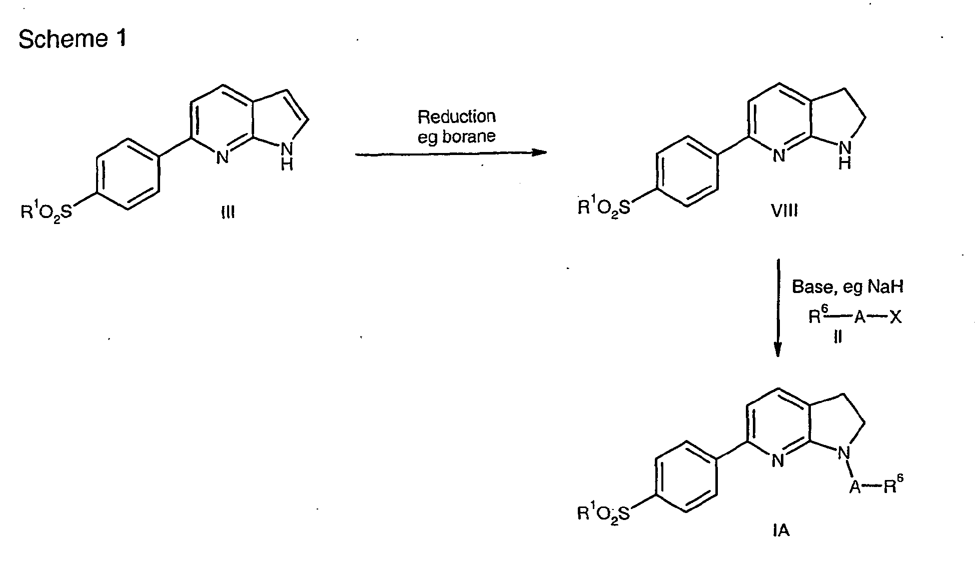

Conveniently, the conversion of compounds of formula (III) to compounds of formula (VIII) is carried out using a reducing agent, such as borane in the presence of a solvent, such as tetrahydrofuran, and at between ambient and elevated temperature.

Compounds of formula (IB), where each of R⁴ and R⁵ is hydrogen may be prepared by a process which comprises:
reacting a compound R⁶-A-X (II) or a protected derivative thereof, with a compound of formula (III) 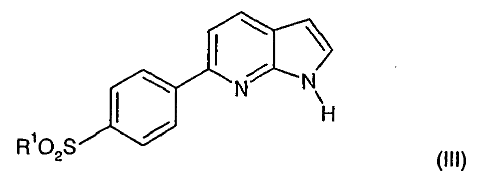 where X is a halogen, such as Cl, Br or I, or a sulfonate, such as methanesulfonate, (4-methyl)benzenesulfonate or trifluoromethanesulfonate, and R⁶ and A are as hereinbefore defined, to produce a compound of formula (IB) in accordance with the present invention : 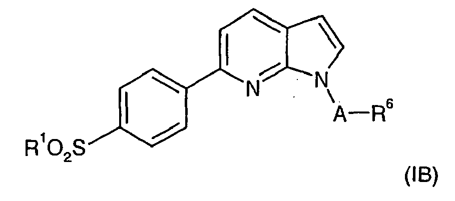
and thereafter and if necessary,
interconverting said compound of formula (IB) into another compound of formula (I); and/or
deprotecting a protected derivative of compound of formula (IB).

The overall synthesis of a compound of formula (IB) is shown in Scheme 2 below, in which R¹, R⁶ and A are as defined above unless otherwise stated; X is a halogen, such as Cl, Br or I, or a sulfonate, such as methanesulfonate, (4-methyl)benzenesulfonate or trifluoromethanesulfonate; and M represents a boronic acid or ester, such as B(OH)₂ or B(OC₁₋₆alkyl)₂ or B(OC(CH₃)₂C(CH₃)₂O).

Referring to Scheme 2, compounds of formula (IB), wherein each of R⁴ and R⁵ are hydrogen and n is 1, 2 or 3, may be prepared by the treatment of compounds of formula (III) with R⁶-A-X (II) in the presence of a base, such as sodium hydride. The reaction is conveniently carried out in a solvent, such as DMF (N,N-dimethylformamide) and at between 0ºC and elevated temperature.

Alternatively, when n is zero the treatment of compounds of formula (III) with R⁶⁻A-X (II) may be carried out in the presence of a catalytic quantity of a copper (I) salt, such as copper (I) iodide, a diamine ligand, such as N,N'-dimethylethylene diamine, and a base, such as potassium phosphate. The reaction is conveniently carried out in a solvent, such as toluene, and at elevated temperature, according, for example, to the method described by Antilla et al in J. C. Antilla, A. Klapars & S. L. Buchwald in J. *Am. Chem. Soc.* **2002,** *124,* 11684.

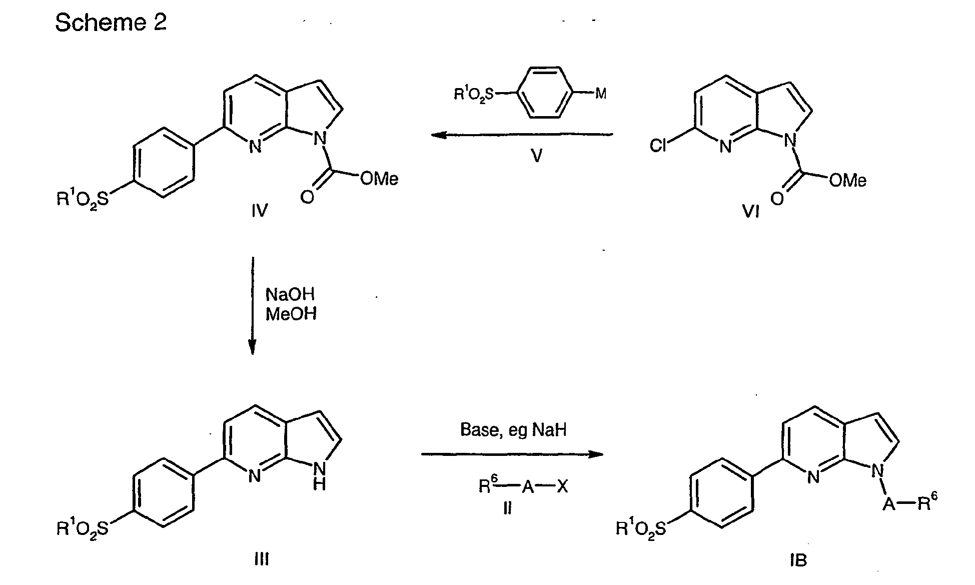

Alternatively when n is zero, the treatment of compounds of formula (III) with R⁶⁻A-X (II) may be carried out in the presence of a catalytic quantity of a palladium source, such as tris(dibenzylidineacetone)dipalladium [Pd₂(dba)₃], a ligand, such as 2-dicyclohexylphosphino-2'-(N,N-dimethylamino)biphenyl, and a base, such as sodium tertbutoxide. The reaction is conveniently carried out in a solvent, such as toluene, and at elevated temperature, according, for example, to the method described by Old et al in D. W. Old, M. C. Harris & S. L. Buchwald in *Org Left* **2000,** *2,* 1403.

The conversion of compounds of formula (IV) to compounds of formula (III) is conveniently carried out using aqueous sodium hydroxide in a solvent, such as an alcohol (e.g. methanol) and at an elevated temperature.

The compound of formula (VI) may be converted to compounds of formula (IV) via a Suzuki coupling reaction employing a palladium source, such as palladium tetrakistriphenylphosphine [Pd(PPh₃)₄], or tris(dibenzylidineacetone)dipalladium [Pd₂(dba)₃] and a ligand, such as triphenylphosphine or tri(tertbutyl)phosphine, and a base, such as sodium carbonate or potassium fluoride, in a solvent such as a water/toluene mix, a water/dimethoxyethane mix or 1,4-dioxane.

Compound (VI) may conveniently be prepared from 7-azaindole according to the method described by S. Minakata, M. Komatsu & Y Ohshiro in *Synthesis* **1992***,* 661.

Compounds of formula (IB), in which both R⁴ and R⁵ are hydrogen, may be converted into other compounds of formula (IB) in accordance with the invention in which R⁵ is other than hydrogen, in accordance with Scheme 3 below:

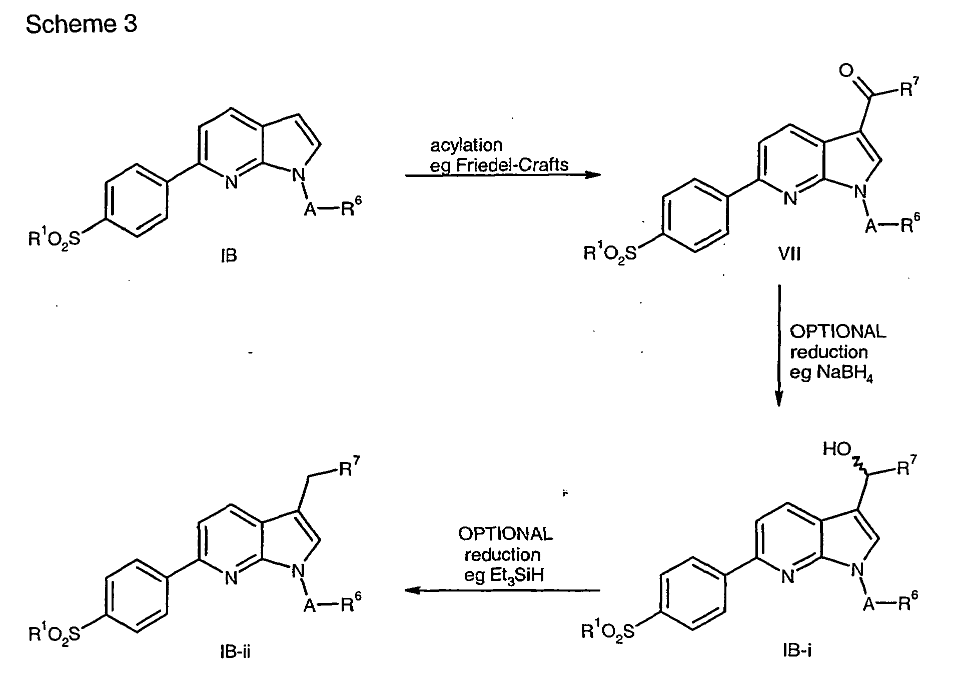

In Scheme 3 R⁷ is branched or linear C₁₋₅alkyl which is unsubstituted or is substituted one or more times by one or more substituents selected from halo and hydroxy such that each of the compounds, except compound of formula (VII), is a compound of formula (IB) in accordance with the present invention.

The conversion of compounds of formula (IB)-i to compounds of formula (IB)-ii, is conveniently carried out using a reducing agent, such as triethylsilane, in the presence of an acid, such as trifluoroacetic acid. The reaction is conveniently carried out in a solvent, such as dichloromethane, and at between 0ºC and ambient temperature.

Conveniently the conversion of compounds of formula (VII) to compounds of formula (IB)-i is carried out using a reducing agent, such as sodium borohydride, in a solvent, such as a methanol/THF mix, and at between 0ºC and ambient temperature.

Compounds of formula (IB), wherein R⁴ and R⁵ are both hydrogen, may be converted to compounds of formula (VII) using a Friedel-Crafts acylation reaction, employing an acid chloride of formula R⁷COCl, in the presence of a catalyst, such as aluminium trichloride. The reaction is conveniently carried out in the presence of a solvent, such as dichloromethane and at between 0ºC and ambient temperature.

In Scheme 3, when R⁷ is hydrogen, the compound of formula (VII) is a compound of formula (IB) according to the present invention. The conversion of compounds of formula (IB), wherein R⁴ and R⁵ are both hydrogen, to compounds of formula (IB), wherein R⁵ is CHO, may be conveniently carried out using a mixture of phosphorus oxychloride and dimethylformamide and at between ambient and elevated temperature (e.g. elevated temperature). Further conversion of compounds of formula (IB), wherein R⁵ is CHO, to compounds of formula (IB), wherein R⁵ is CH₂OH or CH₃ can be carried out in an analogous manner to that described for the conversion of compounds of formula (VII) to compounds of formula (IB)-i, and for the conversion of compounds of formula (IB)-i to compounds of formula (IB)-ii as described above.

It will be appreciated by those skilled in the art that certain of the procedures described in any of Schemes 1 to 3 for the preparation of compounds of the formula (I) or intermediates thereto may not be applicable to some of the possible substituents.

It will be further appreciated by those skilled in the art that it may be necessary to carry out the transformations described in any of Schemes 1 to 3 in a different order from that described, or to modify one or more of the transformations, to provide the desired compound of formula (I).

As will be appreciated by those skilled in the art it may be necessary or desirable at any stage in the synthesis of compounds of formula (I) to protect one or more sensitive groups in the molecule so as to prevent undesirable side reactions. The protecting groups used in the preparation of compounds of formula (I) may be used in conventional manner. See, for example, those described in 'Protective Groups in Organic Synthesis' by Theodora W. Greene and Peter G. M. Wuts, third edition, (Wiley, 1999), incorporated herein by reference, which also describes methods for the removal of such groups.

Compounds of formula R⁶-A-X are either known compounds or may be prepared by literature methods, such as those described in 'Comprehensive Organic Transformations: a guide to functional group preparations' by Richard Larock (VCH, 1989), incorporated herein by reference.

Certain intermediates described above are novel compounds, and it is to be understood that all novel intermediates herein form further aspects of the present invention. Compounds of formulae (III) and (IV) are key intermediates and represent respective particular aspects of the present invention. Methods for the production of these intermediates, as described and defined herein, also form part of the present invention.

Conveniently, compounds of the invention are isolated following work-up in the form of the free base. Pharmaceutically acceptable addition salts of the compounds of the invention may be prepared using conventional means.

Solvates (e.g. hydrates) of a compound of the invention may be formed during the work-up procedure of one of the aforementioned process steps.

The Intermediates and Examples that follow illustrate the invention but do not limit the invention in any way. All temperatures are in °C. Silica chromatography refers to either flash column chromatography performed using Biotage column chromatography cartridges or Solid Phase Extraction (SPE) chromatography, using Varian Mega Bond Elut (Si) cartridges (Anachem) under 15mmHg. Thin layer chromatography (Tlc) was carried out on silica plates. Analytical HPLC was conducted on a Supelcosil LCABZ+PLUS column (3.3 cm x 4.6 mm ID) eluting with 0.1 % HCO₂H and 0.01 M ammonium acetate in water (solvent A), and 0.05% HCO₂H 5% water in acetonitrile (solvent B), using the following elution gradient 0-0.7 minutes 0%B, 0.7-4.2 minutes linear gradient to 100%B, 4.2-5.3 minutes 0%B, 5.3-5.5 minutes 0%B at a flow rate of 3 ml/minutes. The mass spectra (MS) were recorded on a Waters ZQ mass spectrometer using electrospray positive [(ES+ve to give MH+ and M(NH4)+ molecular ions] or electrospray negative [(ES-ve to give (M-H)-- molecular ion] modes. Nuclear magnetic resonance (NMR) spectra were recorded using a Bruker DPX400 spectrometer in CDCl₃ unless stated otherwise. Mass-directed preparative HPLC was conducted on a Supelco ABZ+ column (10cm x 10mm ID, 5µm) eluting with 0.1% HCO₂H in water (solvent A), and 0.05% HCO₂H / 5% water in acetonitrile (solvent B), using the following 10-minute elution gradients according to the analytical LC retention time: 1.5-2.2mins, 0-30%B; 2.0-2.8mins, 5-30%B; 2.5-3.0mins, 15-55%B; 2.8-4.0mins, 30-80%B; 3.8-5.5mins, 50-90%B. The mass spectra (MS) were recorded on a Micromass ZMD mass spectrometer using electrospray positive [(ES+ve to give MH+ and M(NH4)+ molecular ions] or electrospray negative [(ES-ve to give (M-H)-- molecular ion] modes.

In addition to those already defined, the following abbreviations are used: Me, methyl; NMP, N- methyl pyrrolidinone; and THF, tetrahydrofuran.

### Intermediate 1

### Methyl 6-[4-(methylsulfonyl)phenyl]-7-azaindole-1-carboxylate

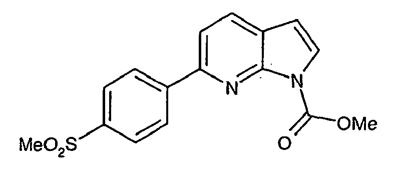

To a thoroughly degassed suspension of compound VI (1.02g, 4.9mmol), obtained for example from 7-azaindole according to the method described by S Minakata, M Komatsu and Y Ohshiro in Synthesis 1992, 661, 4-(methylsulfonyl)phenylboronic acid (1.9g, 9.7mmol) and potassium fluoride (0.93g, 16mmol) in 1,4-dioxane (25mL) at room temperature and under an atmosphere of nitrogen, was added tris(dibenzylidineacetone)dipalladium (67mg) and tritertbutylphosphonium tetrafluoroborate (64mg). After stirring at 100ºC for 14h, the reaction was cooled, concentrated and the residue partitioned between saturated aqueous sodium bicarbonate and ethyl acetate. The phases were separated, the aqueous further extracted with ethyl acetate, and the combined organic phases dried over sodium sulfate, filtered and concentrated *in vacuo* to give, after purification by silica chromatography, eluting with a gradient of cyclohexane to ethyl acetate, the title compound (1.39g, TLC R_{F} 0.26, 2:3 ethyl acetate:cyclohexane) MS m/z 331 (MH⁺).

### Intermediate 2

### 6-[4-(Methylsulfonyl)phenyl]-7-azaindole

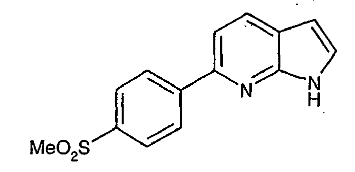

A solution of intermediate 1 (1.34g, 4.0mmol) in 2N aqueous sodium hydroxide (20mL) and methanol (20mL) was heated at 80ºC for 3h. After cooling, the methanol was removed *in vacuo,* the remaining suspension diluted with water and extracted with ethyl acetate. The combined organic phases were dried over sodium sulfate, filtered and concentrated *in vacuo* to give the title compound (1.01g) LC retention time 2.82mins, MS m/z 273 (MH⁺).

### Example 1

### 1-(cyclohexanemethyl)-6-[4-(methylsulfonyl)Phenyl]-7-azaindole

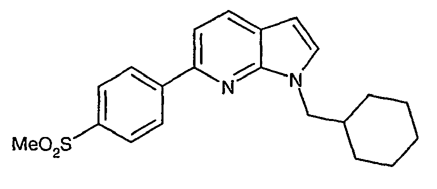

To a suspension of sodium hydride (5mg of a 60% dispersion in mineral oil, 0.12mmol) in dimethylformamide (0.5mL) at 0ºC was added a solution of intermediate 2 (30mg, 0.11 mmol) in dimethylformamide (0.5mL). After stirring at this temperature for 30mins, cyclohexanemethyl bromide (0.017mL, 0.12mmol) was added, and the reaction warmed to room temperature. After stirring for 14h, the solvent was removed *in vacuo,* the residue partitioned between dichloromethane and saturated aqueous sodium carbonate. The organic phase was separated, dried and directly purified by silica chromatography, eluting with dichloromethane, to give the title compound (37mg) LC retention time 4.02mins, MS m/z 369 (MH⁺).

### Example 2

### 1-Phenyl-6-[4-(methylsulfonyl)phenyl]-7-azaindole

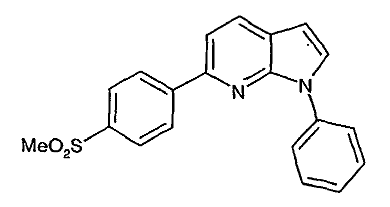

A suspension of intermediate 2 (30mg, 0.11mmol), iodobenzene (0.01mL, 0.09mmol), copper (I) iodide (1mg), N,N'-dimethylethylenediamine (0.002mL) and potassium phosphate (41 mg, 0.19mmol) in toluene (1 mL) was thoroughly degassed and heated at 110ºC, under an atmosphere of nitrogen, for 14h. After cooling, the solvent was removed *in vacuo* and the residue purified by silica chromatography, eluting with dichloromethane, to give the title compound (15mg) LC retention time 3.54mins, MS m/z 349 (MH⁺).

### Intermediate 3

### 1-{1-(cyclohexanemethyl)-6-[4-(methylsulfonyl)phenyl]-7-azaindol-3-yl}-2-methyl-1-propanone

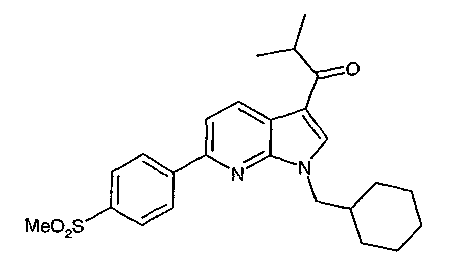

To a stirred suspension of aluminium trichloride (180mg, 1.36mmol) in dichloromethane (5mL) at room temperature was added a solution of example 1 (100mg, 0.27mmol) in dichloromethane (2mL). After stirring for 1.5h, isobutyryl chloride (0.14mL, 1.36mmol) was added and the reaction stirred for a further 6h before being quenched by the addition of methanol. The solvents were removed *in vacuo* and the residue partitioned between dichloromethane and water. The organic phase was separated, dried and directly purified by silica chromatography, eluting with 1:1 dichloromethane: ethyl acetate, to give the title compound (46mg) LC retention time 3.85mins, MS m/z 439 (MH⁺).

### Intermediate 4

### 1-{1-(cyclohexanemethyl)-6-[4-(methylsulfonyl)phenyl]-7-azaindol-3-yl}-2-methyl-1-propanol

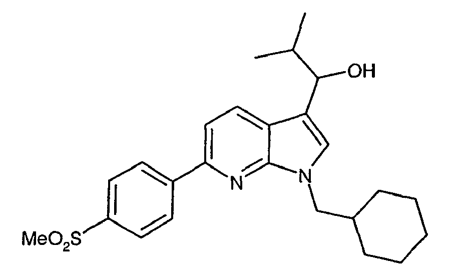

To a stirred solution of intermediate 3 (40mg, 0.09mmol) in a methanol/THF mix (1:1, 4mL) at 0ºC was added sodium borohydride (7mg, 0.18mmol). The reaction was warmed to room temperature and stirred for 3h. Water was added, and the mixture extracted with dichloromethane. The combined organic layers were dried and concentrated to give the title compound (33mg) LC retention time 3.90mins, MS m/z 441 (MH⁺)

### Example 3

### 1-(cyclohexanemethyl)-3-(2-methylprop-1-yl)-6-[4-(methylsulfonyl)phenyl]-7-azaindole

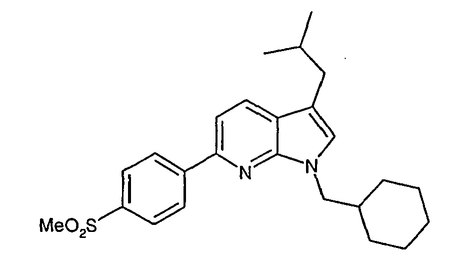

To a stirred solution of intermediate 4 (18mg, 0.041 mmol) in dichloromethane (1 mL) were added triethylsilane (0.008mL, 0.053mmol) and trifluoroacetic acid (0.016mL, 0.20mmol). After stirring for 3h, water was added, the mixture separated and the aqueous phase further extracted with dichloromethane. The combined organic layers were dried, concentrated and purified by mass-directed preparative HPLC to give the title compound (6mg) LC retention time 4.29mins, MS m/z 425 (MH⁺)

### Example 4

### 1-(cyclohexanemethyl)-6-[4-(methylsulfonyl)phbnyl]-7-azaindole-3-carboxaldehyde

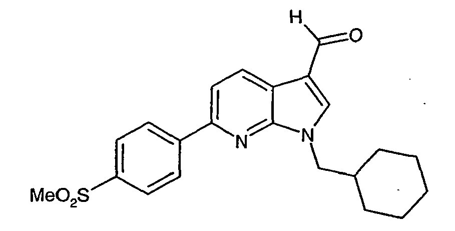

Dimethyformamide (1 mL) was added to phosphorus oxychloride (0.07mL, 0.75mmol) at 0ºC. After stirring for 10min at room temperature, the reaction was cooled to 0ºC before a solution of example 1 (250mg, 0.68mmol) in dimethyformamide (2mL) was added. The reaction was heated at 60ºC for 2h before being cooled and quenched by the addition of saturated aqueous potassium carbonate (25mL) and extracted with dichloromethane. The combined organic layers were dried and concentrated to give, after purification by silica chromatography eluting with 1:1 dichloromethane: ethyl acetate, the title compound (228mg) LC retention time 3.58mins, MS m/z 397 (MH⁺).

### Example 5

### {1-(cyclohexanemethyl)-6-[4-(methylsulfonyl)phenyl]-7-azaindol-3-yl}methanol

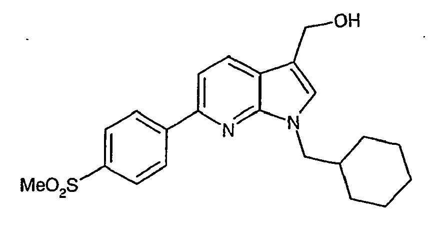

To a stirred suspension of example 4 (100mg, 0.25mmol) in a methanol/THF mix (1:1, 5mL) at 0ºC was added sodium borohydride (19mg, 0.50mmol). The reaction was warmed to room temperature and stirred for 3h. Water was added, and the mixture extracted with ether. The combined organic layers were dried and concentrated to give the title compound (96mg) LC retention time 3.42mins, MS m/z 399 (MH⁺).

### Example 6

### 1-(cyclohexanemethyl)-3-methyl-6-[4-(methylsulfonyl)phenyl]-7-azaindole

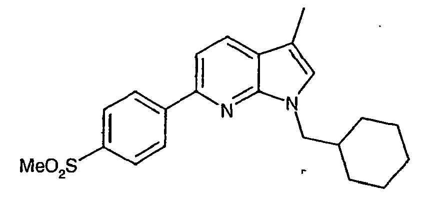

To a stirred solution of example 5 (25mg, 0.063mmol) in dichloromethane (1 mL) were added triethylsilane (0.013mL, 0.082mmol) and trifluoroacetic acid (0.024mL, 0.32mmol). After stirring for 1h, water was added, the mixture separated and the aqueous phase further extracted with dichloromethane. The combined organic layers were dried, concentrated and purified by silica chromatography, eluting with dichloromethane, to give the title compound (12mg) LC retention time 4.19mins, MS m/z 383 (MH⁺).

### Intermediate 5

### 6-[4-(Methylsulfonyl)phenyl]-7-azaindoline

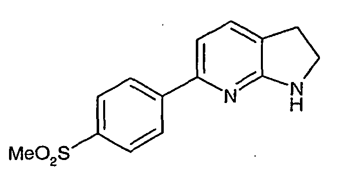

To a stirred solution of intermediate 2 (150mg, 0.55mmol) in tetrahydrofuran (10mL) under an atmosphere of nitrogen was added borane (2.2mL of a 1 M solution in tetrahydrofuran, 2.20mmol). After heating at reflux for 4h, the reaction was cooled to room temperature, further borane (1.1mL of a 1 M solution in tetrahydrofuran, 1.10mmol) was added and heating continued for 2h. The solvent was removed *in vacuo* and the residue purified by silica chromatography, eluting with 1:1 dichloromethane: ethyl acetate, to give the title compound (45mg) LC retention time 2.07mins, MS m/z 275 (MH⁺).

### Example 7

### 1-Benzyl-6-[4-(Methylsulfonyl)phenyl]-7-azaindoline

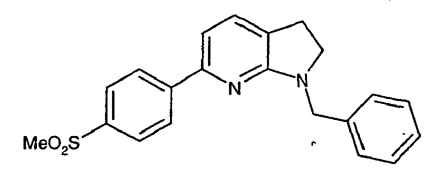

To a suspension of sodium hydride (4mg of a 60% dispersion in mineral oil, 0.10mmol) in dimethylformamide (0.5mL) at 0ºC was added a solution of intermediate 5 (22mg, 0.08mmol) in dimethylformamide (0.5mL). After stirring at this temperature for 30mins, benzyl bromide (0.010mL, 0.08mmol) was added, and the reaction warmed to room temperature. After stirring for 14h, the solvent was removed *in vacuo,* the residue partitioned between dichloromethane and water. The organic phase was separated, dried, concentrated and purified by mass-directed preparative HPLC to give the title compound (9mg) LC retention time 3.66mins, MS m/z 365 (MH⁺).

### Examples 8 to 18

• Examples 8 to 18, as shown in Table 1 that follows, were prepared in the manner described for Examples 1 or 2.

**Table 1**

| | | | | |
|---|---|---|---|---|
| 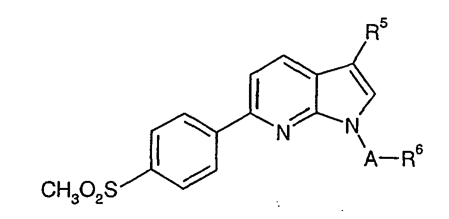 | | | | |

| Ex | A | R⁵ | R⁶ | Mass spec (MH⁺) |
|---|---|---|---|---|
| 8 | CH₂ | H | 2-methylpyrid-3-yl | 378 |
| 9 | SO₂ | H | phenyl | 413 |
| 10 | CH₂ | H | pent-3-yl | 357 |
| 11 | (CH₂)₂ | H | ethyl | 329 |
| 12 | CH₂ | H | phenyl | 363 |
| 13 | | H | 4-fluorophenyl | 367 |
| 14 | CH₂ | H | pyrid-3-yl | 364 |
| 15 | CH₂ | H | pyrid-4-yl | 364 |
| 16 | CH₂ | H | pyrid-2-yl | 364 |
| 17 | CH₂ | H | 6-methylpyrid-3-yl | 378 |
| 18 | CH₂ | H | 4-chlorophenyl | 396,398 |

### Biological Data

### Microsomal Assay

Inhibitory activity against microsomal h-COX2 was assessed against a microsomal preparation from baculovirus infected SF9 cells. An aliquot of microsomal preparation was thawed slowly on ice and a 1/40,000 dilution prepared from it into the assay buffer (sterile water, degassed with argon containing 100mM HEPES (pH 7.4), 10mM EDTA (pH7.4), 1mM phenol, 1mM reduced glutathione, 20mg/ml gelatin and 0.001 mM Hematin). Once diluted the enzyme solution was then sonicated for 5 seconds (Branson sonicator, setting 4, 1cm tip) to ensure a homogeneous suspension. 155µl enzyme solution was then added to each well of a 96-well microtitre plate containing either 5µl test compound (40x required test concentration) or 5µl DMSO for controls. Plates were then mixed and incubated at room temperature for 1 hour. Following the incubation period, 40µl of 0.5µM arachidonic acid was added to each well to give a final concentration of 0.1µM. Plates were then mixed and incubated for exactly 10 minutes (room temperature) prior to addition of 25µl 1 M HCl (hydrochloric acid) to each well to stop the reaction. 25µl of 1 M NaOH (sodium hydroxide) was then added to each well to neutralise the solution prior to determination of PGE₂ levels by enzyme immunoassay (EIA).

The following examples had IC₅₀ values for inhibition of COX-2 of 0.5µM or less and at least 100 fold selectivity for COX-2 over COX-1, based on comparison of the respective IC₅₀ values.
1, 2, 3, 4, 6, 9, 10, 12, 13, 14, 15, 16, 18.

## Claims

1. A compound of formula (1) 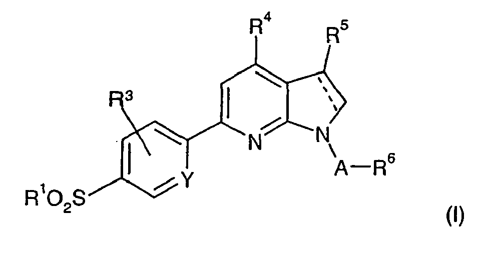 or a pharmaceutically acceptable salt thereof in which:
Y is selected from the group consisting of CH or nitrogen;
R¹ is selected from the group consisting of C₁₋₆alkyl, NH₂ and R²CONH;
R² is selected from the group consisting of H, C₁₋₆alkyl, C₁₋₆alkoxy,
C₁₋₆alkylOC₁₋₆alkyl, phenyl, HO₂CC₁₋₆alkyl, C₁₋₆alkylOCOC₁₋₆alkyl, C₁₋₆alkylOCO, H₂NC₁₋₆alkyl, C₁₋₆alkylOCONHC₁₋₆alkyl and C₁₋₆alkylCONHC₁₋₆alkyl;
R³ is selected from the group consisting of H and halogen;
R⁴ is selected from the group consisting of H, C₁₋₅alkyl, and C₁₋₂alkyl substituted by one to five fluorine atoms;
R⁵ is selected from the group consisting of H, CHO, and C₁₋₆alkyl which is unsubstituted or is substituted one or more times by halogen or hydroxy;
A is (CH₂)ₙ or -SO₂-;
R⁶ is selected from the group consisting of C₁₋₆alkyl, C₄₋₈ cycloalkyl, phenyl and 6-membered heteroaryl, wherein the phenyl and 6-membered heteroaryl ring may be unsubstituted or substituted one or more times by halogen or C₁₋₆ alkyl; and
n is 0 to 3.

2. A compound of formula (IA) 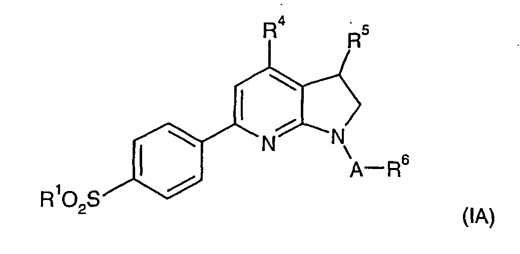 or a pharmaceutically acceptable salt thereof, in which all substituents are as for a compound of formula (I) as defined in claim 1.

3. A compound of formula (IB) 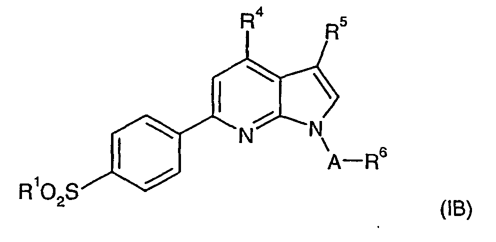 or a pharmaceutically acceptable salt thereof, in which all substituents are as for a compound of formula (I) as defined in claim 1.

4. A compound according to any of claims 1 to 3 wherein R¹ is C₁₋₆alkyl.

5. A compound according to any of claims 1 to 4 wherein R⁴ is H, CHF₂, CH₂F, CF₃ or C₁₋₄alkyl.

6. A compound according to any of claims 1 to 5 wherein R⁵ is H, C₁₋₄alkyl, - CHO, or -(CH₂)ₙCH₂OH.

7. A compound according to any of claims 1 to 6 wherein R⁶ is C₃₋₅alkyl, cyclohexyl, pyridyl optionally substituted by C₁₋₃alkyl, or phenyl optionally substituted by halogen.

8. A compound according to any of claims 1 to 7 wherein n is 0 or 1.

9. A compound according to claim 3 wherein R¹ is C₁₋₃alkyl, R⁴ is H, CHF₂, CH₂F, CF₃ or C₁₋₄alkyl, R⁵ is H, C₁₋₄alkyl, -CHO, or -CH₂OH, n is 1, and R⁶ is C₃₋₅alkyl, cyclohexyl, pyridyl optionally substituted by C₁₋₃alkyl, or phenyl optionally substituted by halogen.

10. A compound according to claim 3 wherein R¹ is C₁₋₃alkyl, R⁴ is H, CHF₂, CH₂F, CF₃ or C₁₋₄alkyl, R⁵ is H, C₁₋₄alkyl, -CHO, or -CH₂OH, n is 0, and R⁶ is phenyl optionally substituted by halogen.

11. A compound according to claim 3 wherein R¹ is CH₃, R³ is H, R⁴ is H, R⁵ is H, C₁₋₄alkyl, -CHO, or -CH₂OH, A is (CH₂)ₙ and n is 1, and R⁶ is C₃₋₅alkyl, cyclohexyl, pyridyl optionally substituted by CH₃, or phenyl optionally substituted by chloro.

12. A compound according to claim 3 wherein R¹ is CH₃, R³ is H, R⁴ is H, R⁵ is H, A is (CH₂)ₙ and n is 0, and R⁶ is phenyl optionally substituted by fluoro.

13. A compound of formula (I) as claimed in claim 1 and selected from any of the Examples 1 to 18.

14. A process for the preparation of compounds of formula (IA), as defined in claim 2, where each of R⁴ and R⁵ is hydrogen, which comprises:
reducing a compound of formula (III) 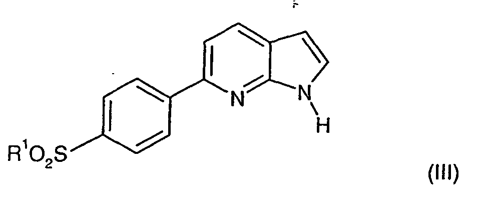
to form a compound of formula (VIII); 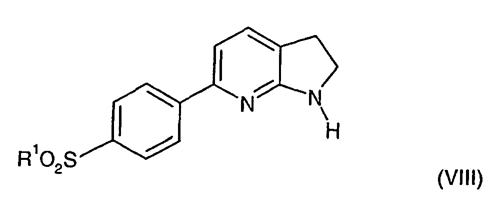
reacting said compound of formula (VIII) with a compound R⁶-A-X, or a protected derivative thereof, where X is a halogen, such as Cl, Br or I, or a sulfonate such as methanesulfonate, (4-methyl)benzenesulfonate or trifluoromethanesulfonate, and A and R⁶ are as hereinbefore defined; such as to produce a compound of formula (IA), wherein R⁴ and R⁵ are both hydrogen 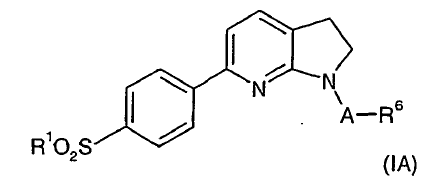
and thereafter and if necessary,
interconverting said compound of formula (IA) into another compound of formula (IA); and/or
deprotecting a protected derivative of compound of formula (IA).

15. A process for the preparation of compounds of formula (IB), as defined in claim 3, where each of R⁴ and R⁵ is hydrogen, which comprises:
reacting a compound R⁶-A-X (II) or a protected derivative thereof, with a compound of formula (III) 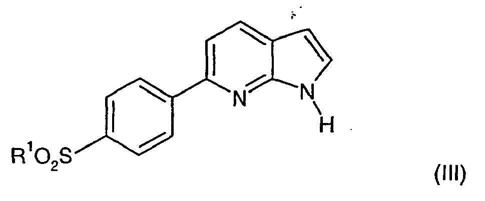
where X is a halogen, such as Cl, Br or I, or a sulfonate, such as methanesulfonate, (4-methyl)benzenesulfonate or trifluoromethanesulfonate, and R⁶ and A are as hereinbefore defined, to produce a compound of formula (IB) in accordance with the present invention : 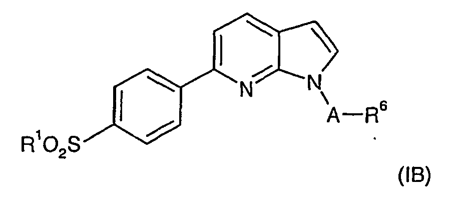 and thereafter and if necessary,
interconverting said compound of formula (IB) into another compound of formula (I); and/or
deprotecting a protected derivative of compound of formula (IB).

16. A pharmaceutical composition comprising a compound of formula (I) as defined in any of claims 1 to 10 in admixture with one or more physiologically acceptable carriers or excipients.

17. A compound of formula (I) as defined in any of claims 1 to 10 for use in human or veterinary medicine.

18. The use of a compound of formula (I) as defined in any of claims 1 to 10 for the manufacture of a therapeutic agent for the treatment of a condition which is mediated by COX-2.

19. The use of a compound of formula (I) as defined in any of claims 1 to 10 for the manufacture of a therapeutic agent for the treatment of an inflammatory disorder.

## Patentansprüche

1. Verbindung der Formel (I) 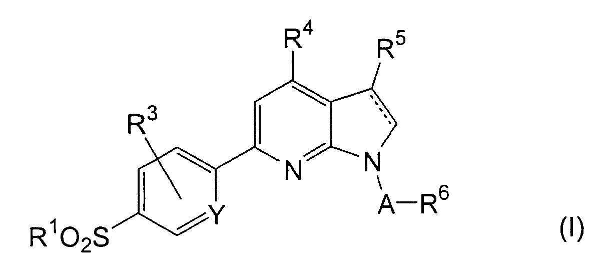 oder ein pharmazeutisch annehmbares Salz davon, in dem:
Y ausgewählt ist aus der Gruppe bestehend aus CH oder Stickstoff;
R¹ ausgewählt ist aus der Gruppe bestehend aus C₁₋₆₋Alkyl, NH₂ und R²CONH;
R² ausgewählt ist aus der Gruppe bestehend aus H, C₁₋₆₋Alkyl, C₁₋₆-Alkoxy, C₁₋₆-AlkylOC₁₋₆-Alkyl, Phenyl, HO₂CC₁₋₆-Alkyl, C₁₋₆-AlkylOCOC₁₋₆-Alkyl, C₁₋₆-AlkylOCO, H₂NC₁₋₆-Alkyl, C₁₋₆-AlkylOCONHC₁₋₆-Alkyl und C₁₋₆₋AlkylCONHC₁₋₆-Alkyl;
R³ ausgewählt ist aus der Gruppe bestehend aus H und Halogen;
R⁴ ausgewählt ist aus der Gruppe bestehend aus H, C₁₋₅₋Alkyl und C₁₋₂-Alkyl, substituiert durch ein bis vier Fluoratome;
R⁵ ausgewählt ist aus der Gruppe bestehend aus H, CHO und C₁₋₆-Alkyl, welches ein oder mehrere Male durch Halogen oder Hydroxy substituiert ist oder nicht substituiert ist;
A (CH₂)ₙ oder -SO₂- ist;
R⁶ ausgewählt ist aus der Gruppe bestehend aus C₁₋₆₋Alkyl, C₄₋₈-Cycloalkyl, Phenyl und einem 6-gliedrigen Heteroaryl, wobei Phenyl und der 6-gliedrige Heteroarlyring nicht substituiert sein können oder mit Halogen oder C₁₋₆-Alkyl ein oder mehrere Male substituiert sein können; und
n 0 bis 3 ist.

2. Verbindung der Formel (IA) 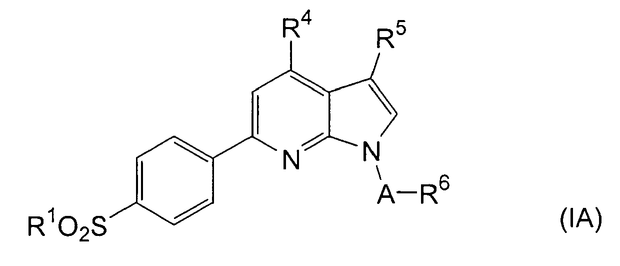 oder ein pharmazeutisch annehmbares Salz davon, in dem alle Substituenten wie bei der Verbindung der Formel (I) sind, die in Anspruch 1 definiert worden ist.

3. Verbindung der Formel (IB) 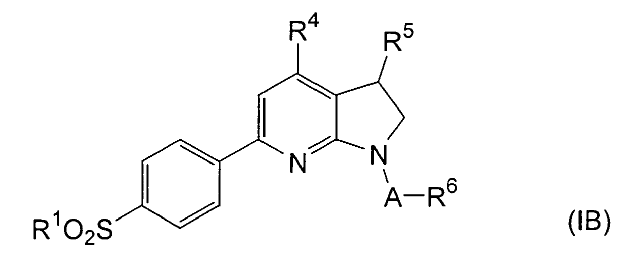 oder ein pharmazeutisch annehmbares Salz davon, in dem alle Substituenten wie bei der Verbindung der Formel (I) sind, wie in Anspruch 1 definiert worden ist.

4. Verbindung gemäß irgendeinem der Ansprüche 1 bis 3, wobei R1 C₁₋₆-Alkyl ist.

5. Verbindung gemäß irgendeinem der Ansprüche 1 bis 4, wobei R⁴ H, CHF₂, CH₂F, CF₃ oder C₁₋₄-Alkyl ist.

6. Verbindung gemäß irgendeinem der Ansprüche 1 bis 5, worin R⁵ H, C₁₋₄-Alkyl, -CHO oder -(CH₂)ₙCH₂OH ist.

7. Verbindung gemäß irgendeinem der Ansprüche 1 bis 6, wobei R⁶ C₃₋₅-Alkyl, Cyclohexyl, Pyridyl, ggf. substituiert mit C₁₋₃-Alkyl, oder Phenyl, ggf. substituiert mit Halogen, ist.

8. Verbindung gemäß irgendeinem der Ansprüche 1 bis 7, wobei n 0 oder 1 ist.

9. Verbindung gemäß Anspruch 3, worin R¹ C₁₋₃-Alkyl ist, R⁴ H, CHF₂, CH₂F, CF₃ der C₁₋₄-Alkyl ist, R⁵ H, C₁₋₄-Alkyl, -CHO oder -CH₂OH ist, n 1 ist und R⁶ C₃₋₅-Alkyl, Cyclohexyl, Pyridyl, ggf. substituiert mit C₁₋₃-Alkyl, oder Phenyl, ggf. substituiert durch Halogen, ist.

10. Verbindung gemäß Anspruch 3, worin R¹ C₁₋₃-Alkyl ist, R⁴ H, CHF₂, CH₂F, CF₃ oder C₁₋₄-Alkyl ist, R⁵ H, C₁₋₄₋Alkyl, -CHO oder -CH₂OH ist, n 0 ist und R⁶ Phenyl ist, ggf. substituiert mit Halogen.

11. Verbindung gemäß Anspruch 3, wobei R¹ CH₃ ist, R³ H ist, R⁴ H ist, R⁵ H, C₁₋₄-Alkyl, -CHO oder -CH₂OH ist, A (CH₂)ₙ ist und n 1 ist, und R⁶ C₃₋₅-Alkyl, Cyclohexyl, Pyridyl, ggf. substituiert durch CH₃, oder Phenyl, ggf. substituiert durch Chlor, ist.

12. Verbindung gemäß Anspruch 3, worin R¹ CH₃ ist, R³ H ist, R⁴ H ist, R⁵ H ist, A (CH₂)ₙ ist und n 0 ist, und R⁶ Phenyl, ggf. substituiert durch Fluor, ist.

13. Verbindung nach Formel (I), wie in Anspruch 1 beansprucht, und ausgewählt aus irgendeinem der Beispiele 1 bis 18.

14. Verfahren zur Herstellung von Verbindungen der Formel (IA), wie in Anspruch 2 definiert, wobei R⁴ und R⁵ jeweils Wasserstoff sind, welches umfasst:
Reduzieren einer Verbindung der Formel (III), 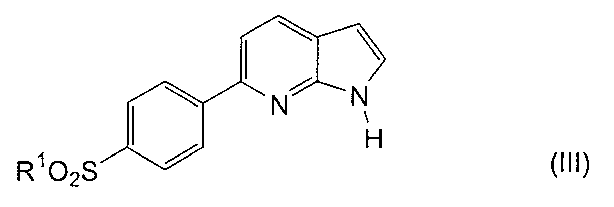
um eine Verbindung der Formel (VIII) zu bilden; 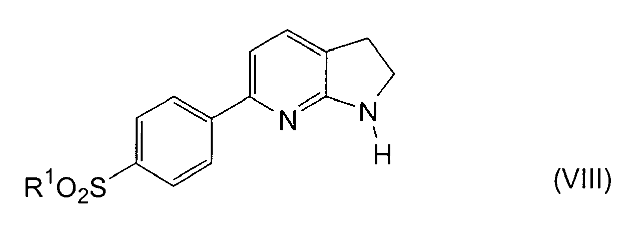
Umsetzen der Verbindung (VIII) mit einer Verbindung R⁶-A-X oder einem geschützten Derivat davon, wobei X Halogen ist, wie Cl, Br oder I, oder ein Sulfonat, wie Methansulfonat, (4-Methyl)benzolsulfonat oder Trifluormethansulfonat, und A und R⁶ wie zuvor definiert sind; um eine Verbindung der Formel (IA) herzustellen, worin R⁴ und R⁵ beide Wasserstoff sind 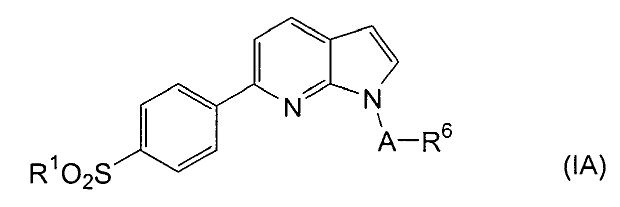
und danach, und falls notwendig,
Umwandeln dieser Verbindung der Formel (IA) in eine andere Verbindung der Formel (IA); und/oder
Entschützen eines geschützten Derivats der Verbindung der Formel (IA).

15. Verfahren zur Herstellung von Verbindungen der Formel (IB) wie in Anspruch 3 definiert, wobei R⁴ und R⁵ jeweils Wasserstoff sind, welches umfasst:
Umsetzen einer Verbindung R⁶-A-X (II) oder eines geschützten Derivats davon mit einer Verbindung der Formel (III) 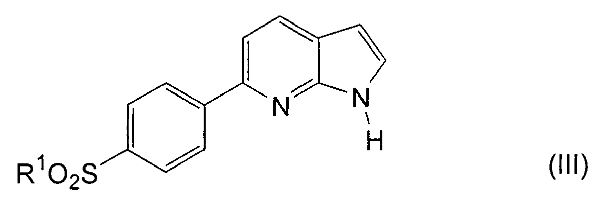 worin X Halogen ist, wie Cl, Br oder I, oder ein Sulfonat, wie Methansulfonat, (4-Methyl)benzolsulfonat oder Trifluormethansulfonat, und R⁶ und A wie zuvor definiert sind, um eine Verbindung der Formel (IB) gemäß der vorliegenden Erfindung herzustellen: 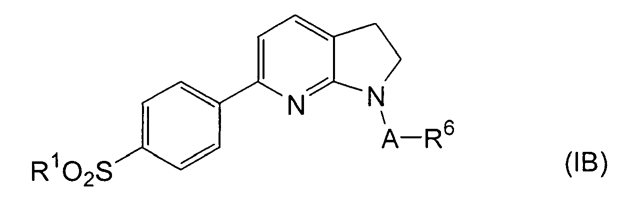
und danach, und falls notwendig,
Umwandeln der Verbindung der Formel (IB) in eine andere Formel der Verbindung der Formel (I); und/oder Entschützen eines geschützten Derivats der Verbindung der Formel (IB).

16. Pharmazeutische Zusammensetzung umfassend eine Verbindung der Formel (I), wie in irgendeinem der Ansprüche 1 bis 10 definiert, in einer Mischung mit einem oder mehreren physiologisch annehmbaren Trägern oder Exzipienten.

17. Verbindung der Formel (I) wie in irgendeinem der Ansprüche 1 bis 10 für Anwendung in der Human- oder Veterinärmedizin.

18. Verwendung einer Verbindung der Formel (I) wie in irgendeinem der Ansprüche 1 bis 10 definiert, zur Herstellung eines therapeutischen Mittels zur Behandlung einer Kondition, die durch COX-2 vermittelt wird.

19. Verwendung einer Verbindung der Formel (I) wie in irgendeinem der Ansprüche 1 bis 10 definiert, zur Herstellung eines therapeutischen Mittels zur Behandlung einer entzündlichen Krankheit.

## Revendications

1. Composé de formule (I) 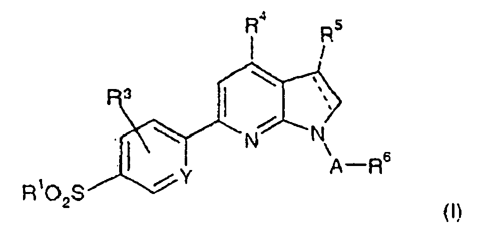 ou sel pharmaceutiquement acceptable de celui-ci, formule dans laquelle :
- Y est choisi au sein du groupe consistant en le groupe CH ou l'azote ;
- R¹ est choisi au sein du groupe consistant en des groupes alkyle C₁₋₆, NH₂ et R²CONH ;
- R² est choisi au sein du groupe consistant en H, et en des groupes alkyle C₁₋₆, alcoxy C₁₋₆, alkyl(C₁₋₆)-O-alkyle C₁₋₆, phényle, HO₂C-alkyle C₁₋₆, alkyl(C₁₋₆)-OCO-alkyle C₁₋₆, alkyl(C₁₋₆,)-OCO, H₂N-alkyle C₁₋₆, alkyl(C₁₋₆)-OCONH-alkyle C₁₋₆ et alkyl(C₁₋₆)-CONH-alkyle C₁₋₆ ;
- R³ est choisi au sein du groupe consistant en H et des halogènes ;
- R⁴ est choisi au sein du groupe consistant en H et en des groupes alkyle C₁₋₅, et alkyle C₁₋₂ substitués par un à cinq atomes de fluor ;
- R⁵ est choisi au sein du groupe consistant en H et en des groupes CHO et alkyle C₁₋₆ qui sont non substitués ou substitués une ou plusieurs fois par un halogène ou un hydroxy ;
- A représente le groupe (CH₂)ₙ ou -SO₂-;
- R⁶ est choisi au sein du groupe consistant en des groupes alkyle C₁₋₆, cycloalkyle C₄₋₈, phényle et hétéroaryle à 6 atomes, où le groupe phényle et le cycle hétéroaryle à 6 atomes peuvent être non substitués ou substitués une ou plusieurs fois par un halogène ou un groupe alkyle C₁₋₆ ; et
- n est de 0 à 3.

2. Composé de formule (IA) 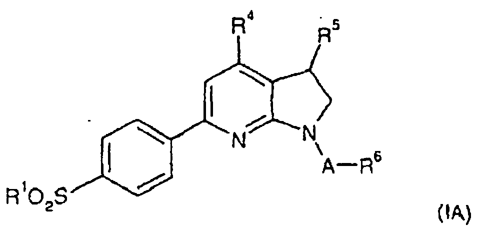 ou sel pharmaceutiquement acceptable de celui-ci, dans laquelle tous les substituants sont tels que définis pour la formule (I) dans la revendication 1.

3. Composé de formule (IB) 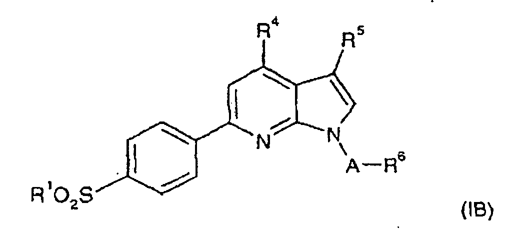 ou sel pharmaceutiquement acceptable de celui-ci, dans laquelle tous les substituants sont tels que définis pour la formule (I) dans la revendication 1.

4. Composé selon l'une quelconque des revendications 1 à 3, dans lequel R¹ représente un groupe alkyle C₁₋₆.

5. Composé selon l'une quelconque des revendications 1 à 4, dans lequel R⁴ représente H, un groupe CHF₂, CH₂F, CF₃ ou alkyle C₁₋₄.

6. Composé selon l'une quelconque des revendications 1 à 5, dans lequel R⁶ représente H, un groupe alkyle C₁₋₄, -CHO ou (CH₂)ₙCH₂OH.

7. Composé selon l'une quelconque des revendications 1 à 6, dans lequel R⁶ représente un groupe alkyle C₃₋₅, cyclohexyle, pyridyle éventuellement substitué par un groupe alkyle C₁₋₃, ou phényle éventuellement substitué par un halogène.

8. Composé selon l'une quelconque des revendications 1 à 7, dans lequel n est égal à 0 ou à 1.

9. Composé selon la revendication 3, dans lequel R¹ représente un groupe alkyle C₁₋₃, R⁴ représente H, un groupe CHF₂, CH₂F, CF₃ ou alkyle C₁₋₄, R⁵ représente H, un groupe alkyle C₁₋₄, -CHO ou CH₂OH, n est égal à 1 et R⁶ représente un groupe alkyle C₃₋₅, cyclohexyle, pyridyle éventuellement substitué par un groupe alkyle C₁₋₃, ou phényle éventuellement substitué par un halogène.

10. Composé selon la revendication 3, dans lequel R¹ représente un groupe alkyle C₁₋₃, R⁴ représente H, un groupe CHF₂, CH₂F, CF₃ ou alkyle C₁₋₄, R⁵ représente H, un groupe alkyle C₁₋₄, -CHO ou CH₂OH, n est égal à 0 et R⁶ représente un groupe phényle éventuellement substitué par un halogène.

11. Composé selon la revendication 3, dans lequel R¹ représente un groupe CH₃, R³ représente H, R⁴ représente H, R⁵ représente H, un groupe alkyle C₁₋₄, -CHO ou CH₂OH, A représente un groupe (CH₂)ₙ et n est égal à 1 et R⁶ représente un groupe alkyle C₃₋₅, cyclohexyle, pyridyle éventuellement substitué par un groupe CH₃, ou phényle éventuellement substitué par un groupe chloro.

12. Composé selon la revendication 3, dans lequel R¹ représente un groupe CH₃, R³ représente H, R⁴ représente H, R⁵ représente H, A représente un groupe (CH₂)ₙ et n est égal à 0, et R⁶ représente un groupe phényle éventuellement substitué par un groupe fluoro.

13. Composé de formule (I) selon la revendication 1, choisi dans l'un quelconque des Exemples 1 à 18.

14. Procédé de préparation de composés de formule (IA) selon la revendication 2, dans lequel chacun de R⁴ et R⁵ représente l'hydrogène, qui comprend :
- la réduction d'un composé de formule (III) 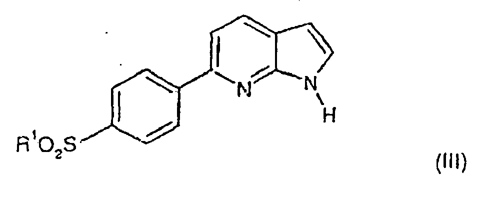 pour former un composé de formule (VIII) : 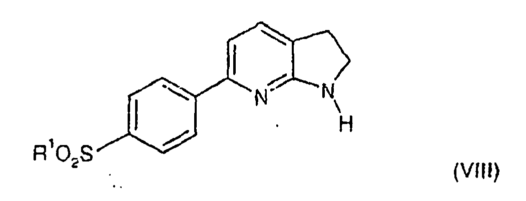
- la réaction dudit composé de formule (VIII) avec un composé R⁶-A-X ou un dérivé protégé de celui-ci où X représente un halogène tel que Cl, Br ou I, ou avec un sulfonate tel qu'un méthanesulfonate, un (4-méthyl)benzènesulfonate ou un trifluorométhanesulfonate, et A et R⁶ sont tels que définis plus haut ; de manière à produire un composé de formule (IA) dans laquelle R⁴ et R⁵ représentent tous deux l'hydrogène 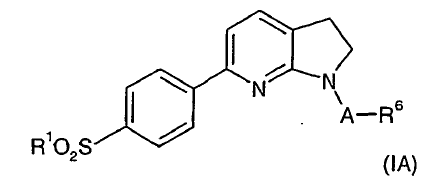 et ensuite, et si nécessaire,
- l'interconversion dudit composé de formule (IA) en un autre composé de formule (IA) ; et/ou
- la suppression de la protection d'un dérivé protégé du composé de formule (IA).

15. Procédé de préparation de composés de formule (IA) selon la revendication 3, dans lesquesl chacun de R⁴ et R⁵ représente l'hydrogène, qui comprend :
- la réaction d'un composé R⁶-A-X ou d'un dérivé protégé de celui-ci, avec un composé de formule (III) 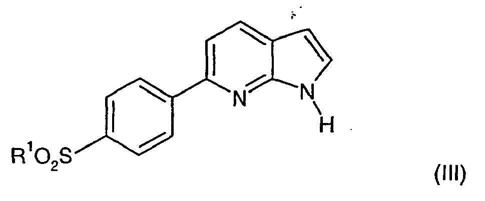 où X représente un halogène tel que Cl, Br ou I ou un sulfonate tel qu'un méthanesulfonate, un (4-méthyl)bebzènesulfonate ou un trifluorométhanesulfonate, et R⁶ et A sont tels que definis plus haut, pour produire un composé de formule (IB) selon la présente invention : 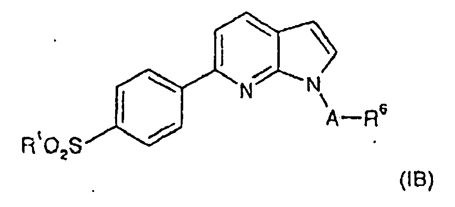 et ensuite, et si nécessaire,
- l'interconversion dudit composé de formule (IB) en un autre composé de formule (I) ; et/ou
- la suppression de la protection d'un dérivé protégé du composé de formule (IB).

16. Composition pharmaceutique comprenant un composé de formule (I) selon l'une quelconque des revendications 1 à 10, en mélange avec un ou plusieurs véhicules ou excipients physiologiquement acceptables.

17. Composé de formule (I) selon l'une quelconque des revendications 1 à 10, en vue de l'utilisation en médecine humaine ou vétérinaire.

18. Utilisation d'un composé de formule (I) selon l'une quelconque des revendications 1 à 10, pour la fabrication d'un agent thérapeutique pour le traitement d'un état qui implique la COX-2.

19. Utilisation d'un composé de formule (I) selon l'une quelconque des revendications 1 à 10, pour la fabrication d'un agent thérapeutique pour le traitement d'un trouble inflammatoire.
